# EUROPEAN PATENT SPECIFICATION

(11) **EP 4 147 677 B1**
(45) Date of publication and mention of the grant of the patent: **26.11.2025**
(21) Application number: 22798678.3
(22) Date of filing: 16.05.2022
(51) Int. Cl.: A61F 2/24

(54) **IMPLANT DELIVERY HANDLE, IMPLANT SYSTEM, AND IMPLANT DELIVERY SYSTEM**
IMPLANTATEINFÜHRUNGSGRIFF, IMPLANTATSYSTEM UND IMPLANTATEINFÜHRUNGSSYSTEM
POIGNÉE DE POSE D'IMPLANT, SYSTÈME D'IMPLANT, ET SYSTÈME DE POSE D'IMPLANT

(30) Priority: 25.08.2021 CN 202110984205
(43) Date of publication of application: 15.03.2023
(73) Proprietor: Shanghai Trulive Medtech Co., Ltd, Shanghai 201206 (CN); Jiangsu Trulive Medtech Co., Ltd, Rudong County Nantong Jiangsu 226400 (CN)
(72) Inventor: ZHANG, Wei, Shanghai 201206 (CN); XU, Haoran, Shanghai 201206 (CN); WEN, Jing, Shanghai 201206 (CN)
(74) Representative: Pohlman, Sandra M.
(86) International application number: PCT/CN2022/092936
(87) International publication number: WO 2022/233338

(56) References cited:
- CN-A- 112 603 599
- CN-A- 112 603 599
- CN-A- 113 648 108
- CN-U- 205 729 576
- CN-U- 209 734 246
- US-A1- 2006 282 150
- US-A1- 2016 242 901
- US-A1- 2017 156 906
- US-A1- 2021 196 494
- US-A1- 2021 196 494

## Description

### TECHNICAL FIELD

The present invention relates to the field of medical devices and, in particular, to an implant delivery handle, an implant system, and an implant delivery system.

### BACKGROUND

Heart valves are flap-like structures in the organs of humans and some animals, which are able to open and close. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent the backward flow of blood.

With the development of social economy and the aging of population, the incidence of valvular heart disease has increased significantly. Studies have shown that this figure has reached up to 13.3% among those 75 years or older. At present, traditional surgical treatment remains first choice for patients with severe valvular disease. However, for those with advanced ages, complications in multiple organs, a history of thoracotomy or poor heart functions, the traditional surgical approach is associated with high risk and high mortality or even precludes some patients.

Interventional valve implantation is a brand new minimally invasive valve replacement technique that has been developed in recent years, which involves loading a valve prosthesis in a delivery system and delivering it into the body of a patient through a catheter. The prosthesis can functionally replace the patient's dysfunctional native valve and improve his/her cardiac condition. This technique is able to treat valve disease without surgically opening the chest or stopping the heartbeat, which may cause significant trauma to the patient.

Due to the high structural complexity of the human heart, accurate valve stent placement is one of the key factors for the success of transcatheter valve replacement (e.g., transcatheter aortic valve replacement (TAVI), transcatheter mitral valve replacement (TMVR), etc.) Accordingly, delivery systems for this purpose are required to allow operations, in particular, guide transmission, self-locking and the like, to be performed in a precise manner. Non-precise operations of such delivery systems may lead to operator mistakes during surgical procedures, inaccurate valve stent placement, or in severe cases, paravalvular leakage, regurgitation or other serious consequences.

From CN 112 603 599 A an implant conveying system is known, which enables to control the speed and distance of implant release at each stage and reduces the risk of release accidents caused by control errors during the implant delivery process.

### SUMMARY OF THE INVENTION

The scope of the present invention is defined by the subject matter of the independent claims. Preferred embodiments are depicted in the dependent claims, the Figures, and the corresponding description.

According to one aspect of the present invention an implant delivery handle according to claim 1 is provided, which is capable of precise operations, which enable guide transmission, self-locking and other operations of an implant delivery system.

According to a further aspect of the present invention an implant system according to claim 9 and a delivery system according to claim 8 are provided, which enable guide transmission, self-locking and other operations of inner and outer tubes, as well as precise operations of the delivery system.

The above objects are attained by an implant delivery handle according to the present invention, which comprises two conveyance sub-systems arranged along an axial direction of an outer tube. The conveyance sub-system located at a distal end is coupled to a proximal end of the outer tube, and the conveyance sub-system located at a proximal end is coupled to a proximal end of an inner tube. Each conveyance sub-system comprises a synchronous conveying device and a ratchet assembly secured to the synchronous conveying device.

The ratchet assembly comprises a cam, a ratchet wheel, a first ratchet pawl and a second ratchet pawl. The ratchet wheel is secured to the synchronous conveying device and is configured to cooperate with the two ratchet pawls to control an axial movement and an axial movement direction of the outer or inner tube to enable a safe self-locking, guide transmission of the outer or inner tube. A first end of each of the two ratchet pawls is rotatably disposed on one side of the cam and a second end of each of the two ratchet pawls is disposed in a vicinity of the ratchet wheel. The two ratchet pawls are configured to control rotation and a rotational direction of the ratchet wheel. The cam is rotatably disposed between the two ratchet pawls and is configured to a control pivoting of the two ratchet pawls.

Optionally, each conveyance sub-system may further comprise a retention mechanism and a handwheel, wherein the retention mechanism is coupled to the synchronous conveying device and to the outer or inner tube, wherein the handwheel is coupled to the synchronous conveying device and is configured to drive an axial movement of the outer or inner tube by the synchronous conveying device and the retention mechanism, and wherein the handwheel and the ratchet assembly are disposed radially on two sides of the synchronous conveying device respectively.

Optionally, the ratchet assembly may further comprise a ratchet wheel housing, a housing cover and a slider, wherein: the ratchet wheel housing is disposed radially on a side of the retention mechanism away from the handwheel; and the ratchet wheel housing is formed in a surface thereof away from the retention mechanism with a depression in which the cam, the ratchet wheel and the two ratchet pawls are accommodated, wherein the housing cover is disposed over the depression, the slider is inserted through the housing cover and is coupled to the cam, wherein the slider is configured to drive the cam to pivot. The first end of each ratchet pawl may be rotatably secured to a side wall of the depression and an outer circumferential surface around the second end of each ratchet pawl is elastically coupled to the side wall of the depression.

Further, the ratchet wheel and the handwheel may be coaxially disposed.

Further, the cam may press one of the ratchet pawls to enable a unidirectional rotation of the ratchet wheel.

Alternatively, the cam may be disposed spacedly between the two ratchet pawls to limit the rotation of the ratchet wheel.

Further, the synchronous conveying device may comprise a driving pulley, a driven pulley and a synchronous conveying belt disposed on the driving and driven pulleys, wherein the driving pulley is disposed coaxially with the handwheel and is configured to rotate with the handwheel to drive the driven and driving pulleys to rotate in a same direction to cause the synchronous conveying belt to move in the axial direction of the outer or inner tube.

Further, the retention mechanism may comprise a catheter holder, a clamp plate fixed portion and a clamp plate, wherein the catheter fixed portion is sleeved over a proximal end portion of the outer or inner tube, wherein the clamp plate fixed portion is secured to the catheter fixed portion and faces toward the synchronous conveying belt, and wherein the clamp plate is retained on the clamp plate fixed portion, with the synchronous conveying belt being clamped between the clamp plate and the clamp plate fixed portion.

In a second aspect, the present invention further provides an implant delivery system, comprising the implant delivery handle as defined above and a catheter assembly. The catheter assembly comprises the outer tube and the inner tube disposed within the outer tube. The implant delivery handle is configured to drive an axial movement of the outer and inner tubes.

In a third aspect, the present invention further provides an implant system comprising the implant delivery handle as defined above, the outer tube, the inner tube, a tapered tip and a retainer. The retainer is disposed at a distal end of the inner tube, and the tapered tip is disposed at a distal end of the outer tube. An implant is crimped within a distal end portion of the outer tube between the tapered tip and the retainer, and the implant delivery handle is configured to drive an axial movement of the outer and inner tubes to enable loading, delivery and release of the implant.

According to an example, not covered by the present invention a method for operating of the implant delivery system as defined above is described. The method comprises:
pivoting the cam to a position where the cam does not press either of the two ratchet pawls on opposite sides of the cam, thereby allowing each ratchet pawl to be limited by the ratchet wheel and enabling a safe self-locking of the inner or outer tube by the synchronous conveying device;
pivoting the cam in a first direction and pressing the first ratchet pawl to cause first ratchet pawl to move out of a tooth gap of the ratchet wheel, thereby allowing the ratchet wheel to rotate in a second direction and enabling guide transmission of the inner or outer tube by the synchronous conveying device; and
pivoting the cam in the second direction and pressing the second ratchet pawl to cause the second ratchet pawl to move out of a tooth gap of the ratchet wheel, thereby allowing the ratchet wheel to rotate in the first direction and enabling guide transmission of the inner or outer tube by the synchronous conveying device,
wherein the first direction is opposite to the second direction, and the conveyance sub-system located at a proximal end is coupled to the inner tube and the conveyance sub-system located at a distal end is coupled to the outer tube.

Compared to the prior art, the present invention offers the following benefits:
The present invention provides an implant delivery handle, an implant system, and a delivery system. A ratchet assembly in the implant delivery handle includes a cam, a ratchet wheel and a first ratchet pawl and a second ratchet pawl. The ratchet wheel is secured to a synchronous conveying device and is configured to cooperate with the two ratchet pawls to control an axial movement and an axial movement direction of the outer or inner tube so as to enable safe self-locking, guide transmission of the inner or outer tube. A first end of each of the ratchet pawls is disposed rotatably at on one side of the cam and a second end of each of the ratchet pawls is disposed in the vicinity of the ratchet wheel, and the two ratchet pawls are configured to control a rotation and a rotational direction of the ratchet wheel. The cam is rotatably disposed between the two ratchet pawls and is configured to control pivoting of the two ratchet pawls. According to the present invention, the ratchet wheel cooperates with the two ratchet pawls to control an axial movement and an axial movement direction of the outer or inner tube so as to enable safe self-locking, guide transmission of the outer or inner tube. In this way, the inner and outer tubes can be unidirectionally locked, allowing a safe self-locking of the implant delivery handle without positional uncontrollability of the inner or outer tube.

The present invention also provides an implant system and a delivery system, which enable precise operation of the implant delivery system and the delivery system, avoid undesirable backward movement of the inner or outer tube due to an operator's operational mistake. In this way, positional uncontrollability of the inner or outer tube can be prevented.

### BRIEF DESCRIPTION OF THE DRAWINGS

Fig. 1 is a schematic perspective view of an implant delivery system according to an embodiment of the present invention;
Figs. 2a to 2b are schematic partial perspective views of an implant delivery handle according to an embodiment of the present invention;
Fig. 3 is a schematic enlarged view of region A of Fig. 1;
Figs. 4a to 4b are schematic diagrams illustrating the structure of a first retention mechanism according to an embodiment of the present invention;
Fig. 5 is a schematic perspective view of a part of Fig. 2b;
Fig. 6a is a schematic perspective view of a first ratchet wheel housing according to an embodiment of the present invention;
Fig. 6b is a schematic perspective view of a first housing cover according to an embodiment of the present invention; and
Figs. 7a to 7c schematically illustrate cooperation of the first ratchet wheel with two ratchet pawls according to an embodiment of the present invention.

### Description of Reference Numerals in Drawings:

1: Catheter Assembly; 110: Inner Tube; 120: Outer Tube; 130: Guidewire Tube; 140: Tapered Tip; 150: Retainer; 2: Implant Delivery Handle; 210: Housing; 220: Securing Rod; 3: Implant;
300: First Conveyance Sub-system; 310: First Retention Mechanism; 311: First Catheter Fixed Portion; 312: First Clamp Plate Fixed Portion; 313: First Clamp Plate; 320: First Synchronous Conveying Device; 321: First Driving Pulley; 3211: First Pulley Body; 3212: First Transmission Shaft; 322: First Driven Pulley; 323: First Synchronous Conveying Belt; 330: First Handwheel; 340: First Ratchet Assembly; 341: First Ratchet Wheel Housing; 3411: First Depression; 3412: First Transmission Shaft Through Hole; 3413: First Guide Member Through Hole; 3414: First Notch; 3415: Second Notch; 3416: First Fastening Through Hole; 342: First Ratchet Wheel; 343: First Slider; 344: First Ratchet Pawl; 3441: First Spring; 345: Second Ratchet Pawl; 3451: Second Spring; 346: First Guide Member; 3461: First Cam; 3462: First Link; 347: First Housing Cover; 3471: Second Transmission Shaft Through Hole; 3472: Second Guide Member Through Hole; 3473: Second Fastening Through Hole;
400: Second Conveyance sub-System; 410: Second Retention Mechanism; 420: Second Synchronous Conveying Device; 421: Second Driving Pulley; 422: Second Driven Pulley; 423: Second Synchronous Conveying Belt; 430: Second Handwheel; 440: Second Ratchet Assembly.

### DETAILED DESCRIPTION

The implant delivery handle, implant system, and delivery system proposed in the present invention will be described in greater detail below. The present invention will be described in greater detail below with reference to the accompanying drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of actual implementations are described in this specification. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects and features of the present invention will become more apparent upon reading the following more detailed description thereof made with reference to the accompanying drawings and particular embodiments. Note that the figures are provided in a very simplified form not necessarily drawn to exact scale and for the only purpose of facilitating easy and clear description of the disclosed embodiments. As used herein, the term "or" is generally employed in the sense of "and/or", unless the context clearly dictates otherwise. As used herein, the terms "inner", "outer" and similar terms are merely illustrative and do not represent the only implementation possible. As used herein, the terms "proximal end" and "distal end" are employed to describe relative orientations, relative positions and directions between components of a medical device or actions thereof, as viewed by a physician operating the device. Without wishing to be limiting, a "proximal end" usually refers to an end closer to the operator, and a "distal end" usually refers to an end closer to the heart of a patient, during normal operation of the medical device.

Further, since the first and second conveyance sub-systems described below are two synchronous conveyance systems substantially of the same structure, except for slight differences, such as different synchronous conveying belt lengths and different sizes of the second catheter fixed portion coupled to the proximal end of the inner tube and the first catheter fixed portion coupled to the proximal end of the outer tube, which, however, indeed, have no impact on functionality of the two sub-systems, only the elements of the first conveyance sub-system are labeled in the annexed figures, and the first conveyance sub-system is described in greater detail than the second conveyance sub-system in the following embodiments.

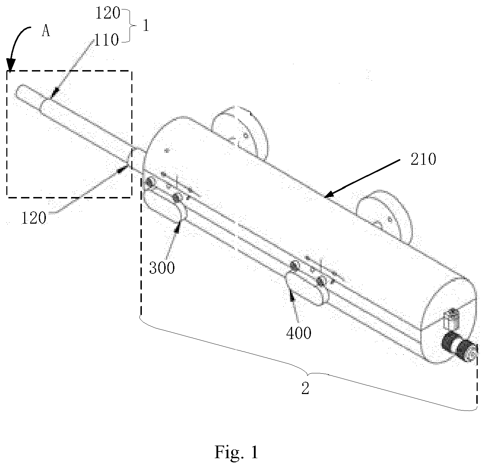

Fig. 1 is a schematic perspective view of an implant delivery system according to an embodiment of the present invention. As shown in Fig. 1, the implant delivery system includes a catheter assembly 1 and an implant delivery handle 2. The implant delivery handle 2 is configured to drive the catheter assembly 1 to move axially.

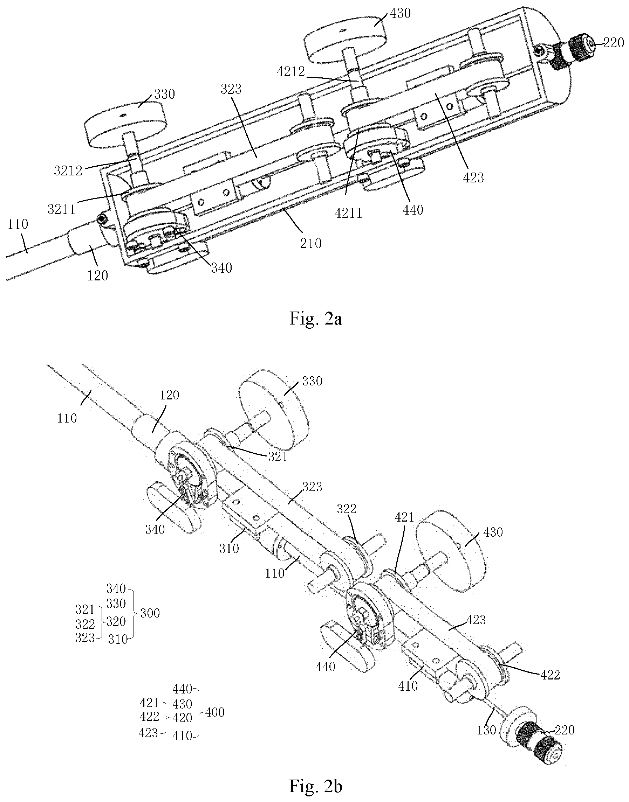

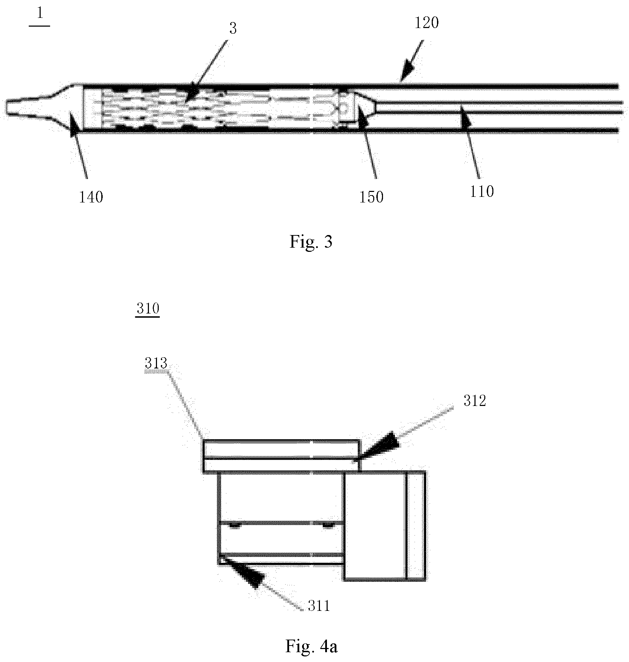

Fig. 2 is a schematic partial perspective view of an implant delivery handle according to an embodiment of the present invention. Fig. 3 is a schematic enlarged view of region A of Fig. 1. As shown in Figs. 2 and 3, the catheter assembly 1 includes an outer tube 120, an inner tube 110 and a guidewire tube 130, which are sequentially sleeved one over another from the outside inward. That is, the outer tube 120 is sleeved over the inner tube 110, which is in turn sleeved over the guidewire tube 130. A guidewire is disposed inside the guidewire tube 130.

The catheter assembly 1 further includes, at a distal end thereof, a tapered tip 140 and a retainer 150. The retainer 150 is disposed at a distal end of the inner tube 110, so that six degrees of freedom of the retainer 150 are all limited. The retainer 150 is provided to retain an implant 3. The tapered tip 140 is detachably disposed at a distal end of the outer tube 120. The implant 3 is located at the distal end of the inner tube 110 and is crimped within a distal end section of the outer tube 120. Before being released, the implant 3 is confined between the tapered tip 140 and the retainer 150. The implant 3 is, for example, a valve stent.

With continued reference to Figs. 2a to 2b, the implant delivery handle 2 includes a housing 210, a securing rod 220, a first conveyance sub-system 300 and a second conveyance sub-system 400. The housing 210 is an enclosed cylindrical housing assembled from parts. The housing 210 has an axial direction that is the same as an axial direction of the outer tube. The housing 210 has a distal end wall and a proximal end wall, each provided therein with a through hole. The through hole in the distal end wall is aligned with the through hole in the proximal end wall. A distal end of the securing rod 220 is secured in the through hole in the proximal end wall and connected to a proximal end of the catheter assembly 1. The catheter assembly 1 passes through the through hole in the distal end wall of the housing 210, so that the distal end of the catheter assembly 1 is located outside the housing 210. An axial direction of the catheter assembly 1 is the same as an axial direction of the housing 210. The first conveyance sub-system 300 and the second conveyance sub-system 400 are of the same structure and are both accommodated for the most part within the housing 210. The housing 210 is configured to protect the first conveyance sub-system 300 and the second conveyance sub-system 400 that are provided therein.

In this embodiment, the distal end of the securing rod 220 is secured to a proximal end of the guidewire tube 130, and both the first conveyance sub-system 300 and the second conveyance sub-system 400 are disposed for the most part within the housing 210 along the axial direction thereof. The first conveyance sub-system 300 is located at a distal end portion of the housing 210, and the second conveyance sub-system 400 is located at a proximal end portion of the housing 210. Moreover, the second conveyance sub-system 400 is coupled to a proximal end of the inner tube 110 and is configured to drive axial movement of the inner tube 110 (from the proximal end to the distal end, or from the distal end to the proximal end) and to control guide transmission of the inner tube 110. The first conveyance sub-system 300 is coupled to a proximal end of the outer tube 120 and is configured to drive axial movement of the outer tube 120 and to control guide transmission of the outer tube 120.

The first conveyance sub-system 300 includes a first retention mechanism 310, a first synchronous conveying device 320, a first handwheel 330 and a first ratchet assembly 340. The first synchronous conveying device 320 includes a first driving pulley 321, a first driven pulley 322 and a first synchronous conveying belt 323 arranged on both the first driving pulley 321 and the first driven pulley 322. The first driving pulley 321 is coupled to the first handwheel 330 such that the first handwheel 330 can drive rotation of the first driving pulley 321, which in turn causes rotation of both the first driven pulley 322 and the first driving pulley 321 in the same direction, as well as causes movement of the first synchronous conveying belt 323 in the axial direction of the housing 210. The first synchronous conveying belt 323 is disposed on one side of (e.g., above) the catheter assembly 1, and the first handwheel 330 is arranged outside the housing 210.

The first driving pulley 321 includes a first pulley body 3211 and a first transmission shaft 3212. The first pulley body 3211 is sleeved over and fixed to the first transmission shaft 3212, and the first transmission shaft 3212 is coupled at one end thereof to the first handwheel 330, thereby allowing the first handwheel 330 to drive rotation of the first transmission shaft 3212 and hence of the first pulley body 3211. The first ratchet assembly 340 is disposed on the side of the first pulley body 3211 away from the first handwheel 330. Preferably, the first synchronous conveying belt 323 is in gear engagement with the first pulley body 3211.

Figs. 4a to 4b are schematic diagrams illustrating the structure of the first retention mechanism in this embodiment. As shown in Figs. 4a to 4b, the first retention mechanism 310 is sleeved over the proximal end of the outer tube 120 and fixed to the first synchronous conveying belt 323. The first retention mechanism 310 includes a first catheter fixed portion 311, a first clamp plate fixed portion 312 and a first clamp plate 313. The first catheter fixed portion 311 is fitted over an outer circumferential surface of a proximal end section of the outer tube 120, and the first clamp plate fixed portion 312 is fixedly attached to a side wall of the first catheter fixed portion 311, and faces toward the first synchronous conveying belt 323. The first clamp plate 313 is retained on the first clamp plate fixed portion 312, with the first synchronous conveying belt 323 being clamped between the first clamp plate 313 and the first clamp plate fixed portion 312. In this way, movement of the first synchronous conveying belt 323 will cause the first retention mechanism 310 to drive axial movement of the outer tube 120. In order for the first synchronous conveying belt 323 to be firmly retained by the first retention mechanism 310, a surface of the first clamp plate 313 facing the first clamp plate fixed portion 312 may be in gear engagement with the first synchronous conveying belt 323.

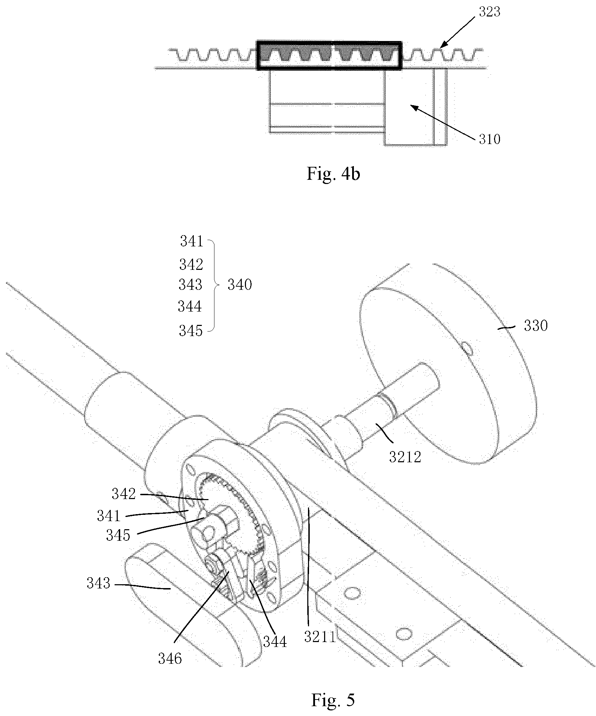

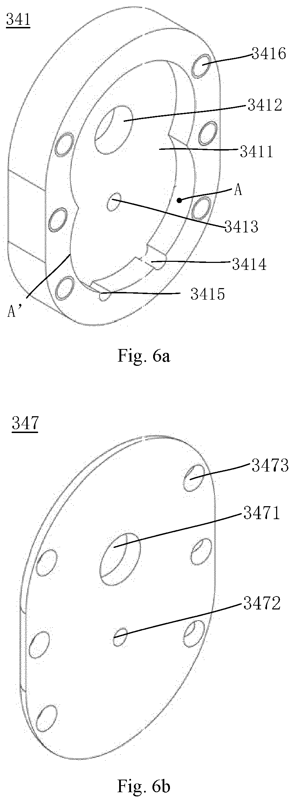

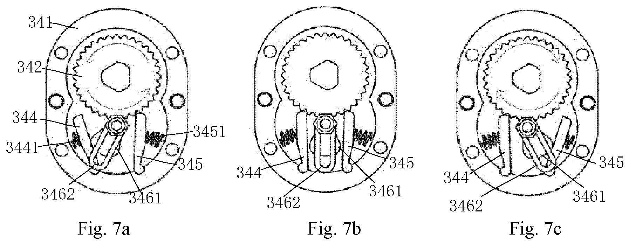

Fig. 5 is a schematic perspective view of a part of Fig. 2b. Fig. 6a is a schematic perspective view of a first ratchet wheel housing in this embodiment. Fig. 6b is a schematic perspective view of a first housing cover in this embodiment. Figs. 7a to 7c schematically illustrate cooperation of the first ratchet wheel with two ratchet pawls in this embodiment. As shown in Figs. 5 to 7c, the first ratchet assembly 340 is secured to an inner wall of the housing 210 away from the first synchronous conveying belt 323. The first ratchet assembly 340 includes a first ratchet wheel housing 341, a first ratchet wheel 342, a first slider 343, a first ratchet pawl 344, a second ratchet pawl 345, a first guide member 346 and a first housing cover 347. The first ratchet wheel housing 341, the first ratchet wheel 342, the first ratchet pawl 344, the second ratchet pawl 345, the first guide member 346 and the first housing cover 347 are all housed in the housing 210, while the first slider 343 is disposed outside the housing 210.

The first ratchet wheel housing 341 is in the shape of a rounded oblong plate and comprises a first depression 3411 in its one surface. The first ratchet wheel 342, the first ratchet pawl 344, the second ratchet pawl 345 and the first guide member 346 are received in the first depression 3411. A first transmission shaft through hole 3412 and a first guide member through hole 3413 are formed in a bottom wall of the first depression 3411 which are spaced apart from each other. The first housing cover 347 has the same shape as the first ratchet wheel housing 341 and is provided as a rounded oblong sheet. The first housing cover 347 is provided with a second transmission shaft through hole 3471 and a second guide member through hole 3472 which are spaced apart from each other. When the first housing cover 347 is disposed in place over the first ratchet wheel housing 341, the first transmission shaft 3212 is aligned with the second transmission shaft through hole 3471, and the end of the first transmission shaft 3212 away from the first handwheel 330 is sequentially inserted through the first pulley body 3211, the first transmission shaft through hole 3412, the first ratchet wheel 342 and the second transmission shaft through hole 3471 and then brought into rotatable abutment against the inner wall of the housing 210. The first ratchet wheel 342 is fixed to the first transmission shaft 3212 so as to be able to rotate with rotation of the first transmission shaft 3212, enabling coaxial rotation of the first ratchet wheel 342 and the first pulley body 3211. The first guide member through hole 3413 is aligned with the second guide member through hole 3472, making it possible for the first guide member 346 to be rotatably secured in the first transmission shaft through hole 3412 and the second guide member through hole 3472. Further, the first slider 343 located outside the housing 210 can be coupled to the first guide member 346.

The first ratchet wheel 342 may be a toothed ratchet wheel. Specifically, it may be a direction-variable ratchet wheel mechanism.

A side wall of the first depression 3411 is provided with a first notch 3414 and a second notch 3415 at locations distant from the first transmission shaft through hole 3412 and symmetric with respect to a line connecting centers of the first transmission shaft through hole 3412 and the first guide member through hole 3413. Moreover, the first notch 3414 and the second notch 3415 are provided on opposite sides of the first guide member 346.

Both the first notch 3414 and the second notch 3415 have axial directions defined along a depth direction of the first depression 3414, and axial cross-sections of the first notch 3414 and the second notch 3415 may be arc-shaped, in particular, semicircular. One end of the first ratchet pawl 344 is rotatably disposed in the first notch 3414, and one end of the second ratchet pawl 345 is rotatably disposed in the second notch 3415.

The first ratchet pawl 344 and the second ratchet pawl 345 are structurally identical and both elongate in shape. The end of the first ratchet pawl 344 away from the first notch 3414 comprises a tooth engageable in a tooth gap of the first ratchet wheel. The end of the second ratchet pawl 345 away from the second notch 3415 comprises a tooth engageable in a tooth gap of the first ratchet wheel.

The first ratchet assembly 340 further includes a first spring 3441 and a second spring 3451. The first spring 3441 has one end coupled to the side wall of the first depression 3411 at a location A and the other end coupled to a surface of the first ratchet pawl 344 facing the first depression 3411. As a result of deformation of the first spring 3441, the first ratchet pawl 344 can swing (pivot) about the first notch 3414 between the first guide member 346 and the first spring 3441 over a smaller angle. The second spring 3451 has one end coupled to the side wall of the first depression 3411 at a location A' and the other end coupled to a surface of the second ratchet pawl 345 facing the first depression 3411. As a result of deformation of the second spring 3451, the second ratchet pawl 345 can swing about the second notch 3415 between the first guide member 346 and the second spring 3451 over a small angle. The locations A and A' are symmetrically located on two sides of a line connecting centers of the first transmission shaft through hole 3412 and the second guide member through hole 3472, and located on two sides of the first notch 3414 and the second notch 3415, and distant from the first transmission shaft through hole 3412.

The first guide member 346 includes a first cam 3461 and a first link 3462, which are secured to each other and rotatably disposed in the first guide member through hole 3413. The first cam 3461 is located between the first guide member through hole 3413 and the first link 3462. Moreover, the first link 3462 and the first cam 3461 are spacedly secured to a shaft of a bolt, which is rotatably secured in the first guide member through hole 3413 and the second guide member through hole, so that the first cam 3461 is located within the first depression, whilst the first link 3462 is located outside the first depression. Additionally, the first housing cover 347 is located between the first ratchet wheel housing 341 and the first link 3462, and two nuts on the bolt are located on opposite sides of the first ratchet wheel housing 341 and the first housing cover. The first cam 3461 is generally in the shape of a rounded triangle and is coupled to the shaft around a corner of the triangle. That is, the joint between the shaft and the first cam is away from a center of the shaft. The first link 3462 is a rounded oblong ring coupled to the shaft around one lengthwise end of the rounded oblong ring.

The first slider 343 includes an elongate first main body, a first coupling member and a first securing member. The first coupling member may be in the form of a rod coupled at its opposite axial ends respectively to the first main body and the first securing member. The other lengthwise end of the first link 3462 is designed to match the shape of the first securing member to allow the first securing member to be fitted in said end of the first link 3462. The first main body is elongate and has an axial direction that is the same as the axial of the housing. The first link 3462 is located between the first cam 3461 and the first slider 343. Movement of the first slider 343 in the axial direction of the housing 210 can cause the first cam 3461 to slightly swing about the first guide member through hole 3413 in the same direction. The first ratchet wheel housing 341 has multiple first fastening through holes 3416 along an edge of the surface facing the first housing cover 347, and the first housing cover 347 has multiple second fastening through holes 3473 along its edge. Each of the first fastening through holes 3416 is aligned with a respective one of the second fastening through holes 3473, and the first housing cover 347 and the first ratchet wheel housing 341 are fastened together by multiple bolts inserted through the respective first fastening through holes 3416 and the respective second fastening through holes 3473. Nuts on these bolts are brought into abutment with the inner wall of the housing 210 facing the first ratchet wheel 342, thereby confining the first ratchet assembly between the inner wall of the housing 210 facing the first ratchet wheel 342 and the side of the first pulley body 3211 away from the first handwheel 330.

The housing 210 has an elongate first slot with an axial direction that is the same as that of the housing 210. The first slot is provided between the first main body and the first securing member, and the first coupling member is inserted in the first slot. Movement of the first main body along the axial direction of the housing will cause the first coupling member to move in the first slot.

When both the first spring 3441 and the second spring 3451 do not elastically deform at all, both the first ratchet pawl 344 and the second ratchet pawl 345 abut against the first ratchet wheel 342, with the tooth of the first ratchet pawl 344 resting in a tooth gap of the first ratchet wheel 342 and the tooth of the second ratchet pawl 345 resting in another tooth gap of the first ratchet wheel 342. As the first ratchet pawl 344 and the second ratchet pawl 345 are located on opposite sides of the first guide member 346, the first ratchet wheel 342 cannot rotate, and the first ratchet assembly 340 is in a self-locking configuration.

When the first slider 343 is moved away from the second ratchet assembly 440, the first cam 3461 will responsively swing about the first guide member through hole 3413 away from the second ratchet assembly 440 (hereinafter, this direction is referred to as the "first direction") to press against the first ratchet pawl 344 to compress the first spring 3441. As a result, the tooth of the first ratchet pawl 344 moves out from the tooth gap of the first ratchet wheel 342 and the tooth of the second ratchet pawl sliding over the tooth gap of the first ratchet wheel, allowing the first ratchet wheel 342 to rotate in a direction opposite to the pivotal direction of the first cam 3461 (i.e., the first direction) ("second direction"). In this way, through the first driving pulley 321, the first handwheel 330 can drive the first synchronous conveying belt 323 and hence the outer tube 120 to move unidirectionally in the axial direction of the housing 210.

When the first slider 343 is moved toward the second ratchet assembly 440, the first cam 3461 will responsively swing about the first guide member through hole 3413 toward the second ratchet assembly 440 (i.e., in the second direction) to press against the second ratchet pawl 345 to compress the second spring 3451. As a result, the tooth of the second ratchet pawl 345 moves out from the tooth gap of the first ratchet wheel 342 and the tooth of the first ratchet pawl sliding over the tooth gap of the first ratchet wheel, allowing the first ratchet wheel 342 to rotate in the (first) direction opposite to the pivotal direction of the first cam 3461 (i.e., the second direction). In this way, through the first driving pulley 321, the first handwheel 330 can drive the first synchronous conveying belt 323 and hence the outer tube 120 to move unidirectionally in the axial direction of the housing 210.

As shown in Fig. 2, the second conveyance sub-system 400 includes a second retention mechanism 410, a second synchronous conveying device 420, a second handwheel 430 and a second ratchet assembly 440. The second synchronous conveying device 420 includes a second driving pulley 421, a second driven pulley 422 and a second synchronous conveying belt 423 arranged on both the second driving pulley 421 and the second driven pulley 422. The second driving pulley 421 is coupled to the second handwheel 430 such that the second handwheel 430 can drive rotation of the second driving pulley 421, which in turn causes rotation of both the second driven pulley 422 and the second driving pulley 421 in the same direction, as well as movement of the second synchronous conveying belt 423 in the axial direction of the housing 210. The second synchronous conveying belt 423 is disposed on one side of (e.g., above) the catheter assembly 1, and the second handwheel 430 is arranged outside the housing 210.

The second driving pulley 421 includes a second pulley body 4211 and a second transmission shaft 4212. The second pulley body 4211 is sleeved over and fixed to the second transmission shaft 4212, and the second transmission shaft 4212 is coupled at one end thereof to the second handwheel 430, thereby allowing the second handwheel 430 to drive rotation of the second transmission shaft 4212 and hence the rotation of the second pulley body 4211. The second ratchet assembly 440 is disposed on the side of the second pulley body 4211 away from the second handwheel 430. Preferably, the second synchronous conveying belt 423 is in gear engagement with the second pulley body 4211.

The second retention mechanism 410 is sleeved over the proximal end of the outer tube 120 and is fixed to the second synchronous conveying belt 423. The second retention mechanism 410 is of the same structure as the first retention mechanism 310 shown in Fig. 4. The second retention mechanism 410 includes a second catheter fixed portion, a second clamp plate fixed portion and a second clamp plate. The second catheter fixed portion is fitted over a proximal end section of the inner tube 110, and the second clamp plate fixed portion is fixedly attached to a side wall of the second catheter fixed portion and faces toward the second synchronous conveying belt. The second clamp plate is retained on the second clamp plate fixed portion, with the second synchronous conveying belt being clamped between the second clamp plate and the second clamp plate fixed portion. In this way, movement of the second synchronous conveying belt will cause the second retention mechanism to drive axial movement of the inner tube 110. In order for the second synchronous conveying belt 423 to be firmly retained by the second retention mechanism 410, a surface of the second clamp plate facing the second clamp plate fixed portion may be in gear engagement with the second synchronous conveying belt 423.

Similarly, the second ratchet assembly 440 is of the same structure as the first ratchet assembly 340, as shown in Figs. 5 to 7c and 2. The second ratchet assembly 440 is secured to the wall of the housing 210 away from the second synchronous conveying belt 423. The second ratchet assembly 440 includes a second ratchet wheel housing, a second ratchet wheel, a second slider, a third ratchet pawl, a fourth ratchet pawl, a second guide member and a second housing cover. The second ratchet wheel housing, the second ratchet wheel, the third ratchet pawl, the fourth ratchet pawl, the second guide member and the second housing cover are all housed in the housing, while the second slider is disposed outside the housing.

The second ratchet wheel housing is in the shape of a rounded oblong plate comprising, in its one surface, a second depression, in which the second ratchet wheel, the third ratchet pawl, the fourth ratchet pawl and the second guide member are received. A third transmission shaft through hole and a third guide member through hole that are spaced apart from each other are formed in a bottom wall of the second depression. The second housing cover has the same shape as the second ratchet wheel housing and is provided as an oblong sheet formed with a fourth transmission shaft through hole and a fourth guide member through hole, which are spaced apart from each other. When the second housing cover is disposed in place over the second ratchet wheel housing, the second transmission shaft is aligned with the fourth transmission shaft through hole, and its end away from the second handwheel 430 is sequentially inserted through the second pulley body, the third transmission shaft through hole, the second ratchet wheel and the fourth transmission shaft through hole and then brought into rotatable abutment against the inner wall of the housing 210. The second ratchet wheel is fixed to the second transmission shaft and is able to rotate with rotation of the second transmission shaft, enabling coaxial rotation of the second ratchet wheel and the second pulley body. The third guide member through hole is aligned with the fourth guide member through hole, making it possible for the second guide member to be rotatably secured in the third transmission shaft through hole and the fourth guide member through hole. Further, the second slider located outside the housing can be coupled to the second guide member.

The second ratchet wheel may be a toothed ratchet wheel. Specifically, it may be a direction-variable ratchet wheel mechanism.

A side wall of the second depression is provided with a third notch and a fourth notch at locations distant from the third transmission shaft through hole and symmetric with respect to a line connecting centers of the third transmission shaft through hole and the third guide member through hole. Moreover, the third and fourth notches are provided on opposite sides of the second guide member.

Both the third and fourth notches have axial directions defined along a depth direction of the second depression and have axial cross-sections, which may be arc-shaped, in particular, semicircular. One end of the third ratchet pawl is rotatably disposed in the third notch, and one end of the fourth ratchet pawl is rotatably disposed in the fourth notch.

The third and fourth ratchet pawls are structurally identical and both elongate in shape. The end of the third ratchet pawl away from the third notch comprises a tooth engageable in a tooth gap of the second ratchet wheel. The end of the fourth ratchet pawl away from the fourth notch comprises a tooth engageable in a tooth gap of the second ratchet wheel.

The second ratchet assembly further includes a third spring and a fourth spring. The third spring is coupled at one end to the side wall of the second depression and at the other end to a surface of the third ratchet pawl facing the second depression. As a result of deformation of the third spring, the third ratchet pawl can swing (pivot) about the third notch between the second guide member and the third spring over a small angle. The fourth spring is coupled at one end to the side wall of the second depression and at the other end to a surface of the fourth ratchet pawl facing the second depression. As a result of deformation of the fourth spring, the fourth ratchet pawl can swing about the fourth notch between the second guide member and the fourth spring over a small angle. The other ends of the third and fourth springs are symmetrically disposed on two sides of a line connecting centers of the third transmission shaft through hole and the fourth guide member through hole, and disposed on two sides of the third and fourth notches, and are distant from the third transmission shaft through hole.

The second guide member includes a second cam and a second link, which are secured to each other and together rotatably disposed in the third guide member through hole, with the second cam being located between the third guide member through hole and the second link. Moreover, the second link and the second cam are spacedly secured to a shaft of a bolt, and the shaft of a bolt is rotatably secured in the third guide member through hole and the second guide member through hole. The second cam is located within the second depression, whilst the second link is located outside the second depression. Additionally, the second housing cover is located between the second ratchet wheel housing and the second link, and two nuts on the bolt are located on opposite sides of the second ratchet wheel housing and the second housing cover. The second cam is generally in the shape of a rounded triangle and is coupled to the shaft around a corner of the triangle. That is, the joint between the shaft and the second cam is away from a center of the second cam. The second link is a rounded oblong ring coupled to the shaft around one lengthwise end of the oblong ring.

The second slider includes an elongate second main body, a second coupling member and a second securing member. The second coupling member may be in the form of a rod coupled at its opposite axial ends respectively to the second main body and the second securing member. The other lengthwise end of the second link is designed to match the shape of the second securing member to allow the second securing member to be fitted in said end of the second link. The first main body is elongate and has an axial direction that is the same as that of the housing. The second link is located between the second cam and the second slider. Movement of the second slider in the axial direction of the housing 210 can cause the second cam to slightly swing about the third guide member through hole. The second ratchet wheel housing has multiple third fastening through holes along an edge of the surface facing the second housing cover, and the second housing cover has multiple fourth fastening through holes along its edge. Each of the third fastening through holes is aligned with a respective one of the fourth fastening through holes, and the second housing cover and the second ratchet wheel housing are fastened together by multiple bolts inserted through the respective third fastening through holes and the respective fourth fastening through holes. Nuts on these bolts are brought into abutment with the inner wall of the housing 210 facing the second ratchet wheel, thereby confining the second ratchet assembly between the inner wall of the housing 210 facing the second ratchet wheel and the side of the second pulley body away from the second handwheel 430.

The housing 210 has an elongate second slot with an axial direction that is the same as that of the housing 210. The second slot is provided between the second main body and the second securing member, and the second coupling member is inserted in the second slot. Movement of the second main body along the axial direction of the housing will cause the second coupling member to move in the second slot.

When both the third and fourth springs do not elastically deform at all, both the third and fourth ratchet pawls abut against the second ratchet wheel, with the tooth of the third ratchet pawl resting in a tooth gap of the second ratchet wheel and the tooth of the fourth ratchet pawl resting in another tooth gap of the second ratchet wheel. As the third and fourth ratchet pawls are located on two sides of the second guide member, the second ratchet wheel cannot rotate, and the second ratchet assembly is in a self-locking configuration.

When the second slider is moved away from the second ratchet assembly 440, the second cam will responsively swing about the third guide member through hole away from the second ratchet assembly 440 (i.e., the first direction) to press against the third ratchet pawl to compress the third spring. As a result, the tooth of the third ratchet pawl moves out from the tooth gap of the second ratchet wheel and the tooth of the fourth ratchet pawl slides over the tooth gap of the second ratchet wheel, allowing the second ratchet wheel to rotate in the (second) direction opposite to the pivotal direction of the second cam (i.e., the first direction). In this way, through the second driving pulley 421, the second handwheel 430 can drive the second synchronous conveying belt 423 and hence the outer tube 120 to move unidirectionally in the axial direction of the housing 210.

When the second slider is moved toward the second ratchet assembly 440, the second cam will responsively swing about the third guide member through hole toward the second ratchet assembly 440 (i.e., in the second direction) to press against the fourth ratchet pawl to compress the fourth spring. As a result, the tooth of the fourth ratchet pawl moves out from the tooth gap of the e second ratchet wheel and the tooth of the third ratchet pawl slides over teeth of the second ratchet wheel, allowing the second ratchet wheel to rotate in the (first) direction opposite to the pivotal direction of the second cam (i.e., the second direction). In this way, through the second driving pulley 421, the second handwheel 430 can drive the second synchronous conveying belt 423 and hence the outer tube 120 to move unidirectionally in the axial direction of the housing 210.

In embodiments of the present invention, there is also provided an implant system including the implant delivery handle 2, the outer tube 120, the inner tube 110, the tapered tip 140 and the retainer 150. The retainer 150 is disposed at a distal end of the inner tube 110, and the tapered tip 140 is disposed at a distal end of the outer tube 120. An implant is crimped within a distal end portion of the outer tube 120 and is located between the tapered tip 140 and the retainer 150. The implant delivery handle 2 is configured to drive axial movement of the outer tube 120 and the inner tube 110 for loading, delivery and release of the implant.

In examples, not covered by the present invention, there is also provided a method for operating of an implant delivery system, comprising:
pivoting a cam until it is not pressed by either of two ratchet pawls on opposite sides of the cam and both the ratchet pawls are stopped by a ratchet wheel, thus allowing a safe self-locking of an inner or outer tube by a synchronous conveying device;
pivoting the cam in a first direction to press one of the ratchet pawls out of a tooth gap of the ratchet wheel, thus allowing rotation of the ratchet wheel in a second direction, guide transmission of the inner or outer tube by the synchronous conveying device; and
pivoting the cam in the second direction to press the other one of the ratchet pawls to separate it from the ratchet wheel, thus allowing rotation of the ratchet wheel in the first direction, guide transmission of the inner or outer tube by the synchronous conveying device,
wherein the first direction is opposite to the second direction, and the conveyance sub-system located at the proximal end is coupled to the inner tube and the conveyance sub-system located at the distal end is coupled to the outer tube.

In greater particularity, during operation of the first ratchet assembly 340, as shown in Fig. 7a, moving the first slider to a distal end to cause the first cam 3461 to pivot in the same direction (i.e., the first direction) over a small angle and by urging the first ratchet pawl 344 to cause the tooth of the first ratchet pawl 344 to move out from a tooth gap of the first ratchet wheel 342. As a result, the first ratchet wheel 342 can rotate in the second direction, while exerting a compressive force on the second spring 345, which causes the tooth of the second ratchet pawl 345 to slide out of a tooth gap where it previously rested. Upon sliding the tooth out, the second ratchet pawl is no longer subject to the compressive force and thus pushed back into the next tooth gap of the first ratchet wheel 342, then it is again pressed by the first ratchet wheel. This process is repeated, allowing the first ratchet wheel to rotate in the second direction to cause the outer tube to move unidirectionally along the axial direction of the housing 210.

As shown in Fig. 7c, moving the first slider to the proximal end to cause the first cam 3461 to pivot in the same direction (i.e., the second direction) over a small angle to press against the second ratchet pawl 345, thereby causing the tooth of the second ratchet pawl 345 to move out from a tooth gap of the first ratchet wheel. As a result, the first ratchet wheel 342 can rotate in the first direction, while exerting a compressive force on the first spring 3441, which causes the tooth of the first ratchet pawl 344 to slide out of a tooth gap where it previously rested. Upon sliding the tooth out, the first ratchet pawl 344 is no longer subject to the compressive force and thus pushed back into the next tooth gap of the first ratchet wheel 342, then it is again pressed by the first ratchet wheel 342. This process is repeated, allowing the first ratchet wheel to rotate in the first direction to cause the outer tube to move unidirectionally along the axial direction of the housing 210.

As shown in Fig. 7b, when the first slider 343 is moved to pivot the first cam 3461 to a position where first cam 3461 does not interact with either of the first ratchet pawl 344 and second ratchet pawl 345, the teeth of the first ratchet pawl 344 and the second ratchet pawl 345 rest in different tooth gaps of the first ratchet wheel 342. Thus, the first ratchet assembly 340 is in a self-locking configuration.

During operation of the second ratchet assembly 440, moving the second slider to the distal end to cause the second cam to pivot in the same direction (i.e., the first direction) over a small angle, to press against the third ratchet pawl, thereby causing the tooth of the third ratchet pawl to move out from a tooth gap of the second ratchet wheel. As a result, the second ratchet wheel can rotate in the second direction, while exerting a compressive force on the fourth spring, which causes the tooth of the fourth ratchet pawl to slide out of a tooth gap where it previously rested. Upon sliding the tooth out, the fourth ratchet pawl is no longer subject to the compressive force and thus pushed back into the next tooth gap of the second ratchet wheel, then it is again pressed by the second ratchet wheel. This process is repeated, allowing the second ratchet wheel to rotate in the second direction to cause the inner tube to move unidirectionally along the axial direction of the housing 210.

Moving the second slider to the proximal end to cause the second cam to pivot in the same direction (i.e., the second direction) over a small angle to urge the fourth ratchet pawl, thereby causing the tooth of the fourth ratchet pawl to move out from a tooth gap of the second ratchet wheel. As a result, the second ratchet wheel can rotate in the first direction, while exerting a compressive force on the third spring, which causes the tooth of the third ratchet pawl to slide out of a tooth gap where it previously rested. Upon sliding the tooth out, the third ratchet pawl is no longer subject to the compressive force and thus pushed back into the next tooth gap of the second ratchet wheel, then it is again pressed by the second ratchet wheel. This process is repeated, allowing the second ratchet wheel to rotate in the first direction to cause the outer tube to move unidirectionally along the axial direction of the housing 210.

When the second slider is moved to pivot the second cam to a position where the second cam does not interact with either of the third and fourth ratchet pawls, the teeth of the third and fourth ratchet pawls rest in different tooth gaps of the second ratchet wheel. Thus, the second ratchet assembly is in a self-locking configuration.

In summary, the present invention provides an implant delivery handle, an implant system, and a delivery system. A ratchet assembly in the implant delivery handle includes a cam, a ratchet wheel and two ratchet pawls. The ratchet wheel is secured to a synchronous conveying device and is configured to cooperate with the two ratchet pawls to control axial movement and an axial movement direction of an outer or inner tube so as to enable safe self-locking, guide transmission of the outer or inner tube. The two ratchet pawls are each disposed rotatably at one end on one side of the cam and at the other end in the vicinity of the ratchet wheel, and are configured to control rotation and a rotational direction of the ratchet wheel. The cam is rotatably disposed between the two ratchet pawls and is configured to control pivoting of the two ratchet pawls. According to the present invention, the ratchet wheel cooperates with the two ratchet pawls to control axial movement and an axial movement direction of the outer or inner tube so as to enable safe self-locking, guide transmission of the outer or inner tube. In this way, the inner and outer tubes can be unidirectionally locked, allowing safe self-locking of the implant delivery handle without positional uncontrollability of the inner or outer tube.

The present invention also provides an implant system and a delivery system, which enable precise operation of the implant delivery system and the delivery system, avoid undesirable backward movement of an inner or outer tube due to an operator's operational mistake. In this way, positional uncontrollability of the inner or outer tube can be prevented.

It is to be noted that, as used herein, the terms "first" and "second" are only meant to distinguish various components, elements, steps, etc. from each other rather than indicate logical or sequential orderings thereof, unless otherwise indicated or specified.

## Claims

1. An implant delivery handle (2), comprising two conveyance sub-systems (300,400) arranged along an axial direction of an outer tube (120), wherein: the conveyance sub-system (300) located at a distal end is coupled to a proximal end of the outer tube (120); the conveyance sub-system (400) located at a proximal end is coupled to a proximal end of an inner tube (110); and each conveyance sub-system (300,400) comprises a synchronous conveying device (320, 420) and a ratchet assembly (340,440) secured to the synchronous conveying device (320,420),
wherein the ratchet assembly (340,440) comprises a cam (3461), a ratchet wheel (342), a first ratchet pawl (344) and a second ratchet pawl (345), wherein the ratchet wheel (342) is secured to the synchronous conveying device (320) and is configured to cooperate with the two ratchet pawls (344, 345) to control an axial movement and an axial movement direction of the outer or inner tube (120, 110) to enable safe self-locking guide transmission of the outer or inner tube (120, 110), wherein: a first end of each of the two ratchet pawls (344, 345) is rotatably disposed on one side of the cam (3461); a second end of each of the two ratchet pawls (344, 345) is disposed in a vicinity of the ratchet wheel (342); and the two ratchet pawls (344, 345) are configured to control a rotation and a rotational direction of the ratchet wheel (342), wherein the cam (3461) is rotatably disposed between the two ratchet pawls (344, 345) and is configured to control a pivoting of the two ratchet pawls (344, 345).

2. The implant delivery handle (2) of claim 1, wherein each conveyance sub-system (300,400) further comprises a retention mechanism (310, 410) and a handwheel (330, 430), wherein the retention mechanism (310, 410) is coupled to the synchronous conveying device (320,420) and to the outer or inner tube (120, 110), wherein the handwheel (330, 430) is coupled to the synchronous conveying device (320,420) and is configured to drive an axial movement of the outer or inner tube (120, 110) by the synchronous conveying device (320,420) and the retention mechanism (310, 410), and wherein the handwheel (330, 430) and the ratchet assembly (340,440) are disposed radially on two sides of the synchronous conveying device (320,420) respectively.

3. The implant delivery handle (2) of claim 2, wherein the ratchet assembly (340,440) further comprises a ratchet wheel housing (341), a housing cover (347) and a slider (343), wherein: the ratchet wheel housing (341) is disposed radially on a side of the retention mechanism (310, 410) away from the handwheel (330, 430); and the ratchet wheel housing (341) is formed in a surface thereof away from the retention mechanism (310, 410) with a depression (3411) in which the cam (3461), the ratchet wheel (342) and the two ratchet pawls (344, 345) are accommodated, wherein the housing cover (347) is disposed over the depression (3411), the slider (343) is inserted through the housing cover (347) and is coupled to the cam (3461), wherein the slider (343) is configured to drive the cam (3461) to pivot, wherein the first end of each ratchet pawl (344, 345) is rotatably secured to a side wall of the depression (3411) and an outer circumferential surface around the second end of each ratchet pawl (344, 345) is elastically coupled to the side wall of the depression (3411).

4. The implant delivery handle (2) of claim 3, wherein the ratchet wheel (342) and the handwheel (330, 430) are coaxially disposed.

5. The implant delivery handle (2) of claim 4, wherein
the cam (3461) presses one of the ratchet pawls (344, 345) to enable a unidirectional rotation of the ratchet wheel (342), or
wherein the cam (3461) is disposed spacedly between the two ratchet pawls (344, 345) to limit the rotation of the ratchet wheel (342).

6. The implant delivery handle (2) of claim 2, wherein the synchronous conveying device (320,420) comprises a driving pulley (321, 421), a driven pulley (322, 422) and a synchronous conveying belt (323, 423) disposed on the driving and driven pulleys (321, 421; 322, 422), wherein the driving pulley (321, 421) is disposed coaxially with the handwheel (330,430) and is configured to rotate with the handwheel (330,430) to drive the driven and driving pulleys (321, 421; 322, 422) to rotate in a same direction to cause the synchronous conveying belt (323, 423) to move in the axial direction of the outer or inner tube (120, 110).

7. The implant delivery handle (2) of claim 2, wherein the retention mechanism (310,410) comprises a catheter fixed portion (311), a clamp plate fixed portion (312) and a clamp plate (313), wherein the catheter fixed portion (311) is sleeved over a proximal end portion of the outer or inner tube (120, 110), wherein the clamp plate fixed portion (312) is secured to the catheter fixed portion (311) and faces toward the synchronous conveying belt (323, 423), and wherein the clamp plate (313) is retained on the clamp plate fixed portion (312), with the synchronous conveying belt (323, 423) being clamped between the clamp plate (313) and the clamp plate fixed portion (312).

8. An implant delivery system, comprising the implant delivery handle (2) of any one of claims 1 to 7 and a catheter assembly (1), wherein the catheter assembly (1) comprises the outer tube (120) and the inner tube (110) disposed within the outer tube (120), wherein the implant delivery handle (2) is configured to drive an axial movement of the outer and inner tubes (120, 110).

9. An implant system, comprising the implant delivery handle (2) of any one of claims 1 to 7, an outer tube (120), an inner tube (110), a tapered tip (140) and a retainer (150), wherein the retainer (150) is disposed at a distal end of the inner tube (110), wherein the tapered tip (140) is disposed at a distal end of the outer tube (120), wherein an implant is crimped within a distal end portion of the outer tube (120) between the tapered tip (140) and the retainer (150), and wherein the implant delivery handle (2) is configured to drive an axial movement of the outer and inner tubes (120, 110) to enable loading, delivery and release of the implant.

## Patentansprüche

1. Implantat-Zuführgriff (2), der zwei entlang einer Axialrichtung eines äußeren Rohrs (120) eingerichtete Transportsubsysteme (300, 400) umfasst, wobei: das an einem distalen Ende befindliche Transportsubsystem (300) mit einem proximalen Ende des äußeren Rohrs (120) gekoppelt ist; das an einem proximalen Ende befindliche Transportsubsystem (400) mit einem proximalen Ende eines inneren Rohrs (110) gekoppelt ist; und jedes Transportsubsystem (300, 400) eine Synchrontransportvorrichtung (320, 420) und eine an der Synchrontransportvorrichtung (320, 420) befestigte Ratschenanordnung (340, 440) umfasst,
wobei die Ratschenanordnung (340, 440) einen Nocken (3461), ein Ratschenrad (342), eine erste Ratschenklinke (344) und eine zweite Ratschenklinke (345) umfasst, wobei das Ratschenrad (342) an der Synchrontransportvorrichtung (320) befestigt ist und dazu konfiguriert ist, mit den beiden Ratschenklinken (344, 345) zusammenzuwirken, um eine axiale Bewegung und eine axiale Bewegungsrichtung des äußeren oder des inneren Rohrs (120, 110) zu steuern, um eine sichere selbsthemmende Transportübertragung des äußeren oder inneren Rohrs (120, 110) zu ermöglichen, wobei: ein erstes Ende jeder der beiden Ratschenklinken (344, 345) drehbar an einer Seite des Nockens (3461) angeordnet ist; ein zweites Ende jeder der beiden Ratschenklinken (344, 345) in der Nähe des Ratschenrads (342) angeordnet ist; und die beiden Ratschenklinken (344, 345) dazu konfiguriert sind, eine Drehung und eine Drehrichtung des Sperrrads (342) zu steuern, wobei der Nocken (3461) drehbar zwischen den beiden Ratschenklinken (344, 345) angeordnet ist und dazu konfiguriert ist, ein Schwenken der beiden Ratschenklinken (344, 345) zu steuern.

2. Implantat-Zuführgriff (2) nach Anspruch 1, wobei jedes Transportsubsystem (300, 400) weiter einen Haltemechanismus (310, 410) und ein Handrad (330, 430) umfasst, wobei der Haltemechanismus (310, 410) mit der Synchrontransportvorrichtung (320, 420) und mit dem äußeren oder dem inneren Rohr (120, 110) gekoppelt ist, wobei das Handrad (330, 430) mit der Synchrontransportvorrichtung (320, 420) gekoppelt ist und dazu konfiguriert ist, eine axiale Bewegung des äußeren oder des inneren Rohrs (120, 110) durch die Synchrontransportvorrichtung (320, 420) und den Haltemechanismus (310, 410) anzutreiben, und wobei das Handrad (330, 430) und die Ratschenanordnung (340, 440) radial an zwei Seiten der Synchrontransportvorrichtung (320, 420) angeordnet sind.

3. Implantat-Zuführgriff (2) nach Anspruch 2, wobei die Ratschenanordnung (340, 440) weiter ein Ratschenradgehäuse (341), eine Gehäuseabdeckung (347) und einen Schieber (343) umfasst, wobei: das Ratschenradgehäuse (341) radial an einer Seite des Haltemechanismus (310, 410) von dem Handrad (330, 430) weg angeordnet ist; und das Ratschenradgehäuse (341) in einer Oberfläche davon, von dem Haltemechanismus (310, 410) weg geformt ist, mit einer Vertiefung (3411), in der der Nocken (3461), das Ratschenrad (342) und die beiden Ratschenklinken (344, 345) aufgenommen sind, wobei die Gehäuseabdeckung (347) über der Vertiefung (3411) angeordnet ist, der Schieber (343) durch die Gehäuseabdeckung (347) eingesetzt und mit dem Nocken (3461) gekoppelt ist, wobei der Schieber (343) dazu konfiguriert ist, den Nocken (3461) zum Schwenken anzutreiben, wobei das erste Ende jeder Ratschenklinke (344, 345) drehbar an einer Seitenwand der Vertiefung (3411) befestigt ist und eine Außenumfangsfläche um das zweite Ende jeder Ratschenklinke (344, 345) elastisch mit der Seitenwand der Vertiefung (3411) gekoppelt ist.

4. Implantat-Zuführgriff (2) nach Anspruch 3, wobei das Ratschenrad (342) und das Handrad (330, 430) koaxial angeordnet sind.

5. Implantat-Zuführgriff (2) nach Anspruch 4, wobei
der Nocken (3461) auf eine der Ratschenklinken (344, 345) drückt, um eine unidirektionale Drehung des Ratschenrads (342) zu ermöglichen, oder
wobei der Nocken (3461) beabstandet zwischen den beiden Ratschenklinken (344, 345) angeordnet ist, um die Drehung des Ratschenrads (342) zu begrenzen.

6. Implantat-Zuführgriff (2) nach Anspruch 2, wobei die Synchrontransportvorrichtung (320, 420) eine Antriebsriemenscheibe (321, 421), eine Abtriebsriemenscheibe (322, 422) und ein synchrones Transportband (323, 423) umfasst, das auf den Antriebs- und der Abtriebsriemenscheiben (321, 421; 322, 422) angeordnet ist, wobei die Antriebsriemenscheibe (321, 421) koaxial zu dem Handrad (330, 430) angeordnet ist und dazu konfiguriert ist, sich mit dem Handrad (330, 430) zu drehen, um die Abtriebs- und die Antriebsriemenscheiben (321, 421; 322, 422) in die gleiche Richtung zu drehen, um das synchrone Transportband (323, 423) zu veranlassen, sich in der Axialrichtung des äußeren oder des inneren Rohrs (120, 110) zu bewegen.

7. Implantat-Zuführgriff (2) nach Anspruch 2, wobei der Haltemechanismus (310, 410) einen Katheter-Festabschnitt (311), einen Klemmplatten-Festabschnitt (312) und eine Klemmplatte (313) umfasst, wobei der Katheter-Festabschnitt (311) über einen proximalen Endabschnitt des äußeren oder inneren Rohrs (120, 110) geschoben ist, wobei der Klemmplatten-Festabschnitt (312) an dem Katheter-Festabschnitt (311) befestigt ist und dem synchronen Transportband (323, 423) zugewandt ist, und wobei die Klemmplatte (313) an dem Klemmplatten-Festabschnitt (312) gehalten wird, wobei das synchrone Transportband (323, 423) zwischen der Klemmplatte (313) und dem Klemmplatten-Festabschnitt (312) eingeklemmt ist.

8. Implantat-Zuführsystem, das den Implantat-Zuführgriff (2) nach einem der Ansprüche 1 bis 7 und eine Katheteranordnung (1) umfasst, wobei die Katheteranordnung (1) das äußere Rohr (120) und das innere Rohr (110), das innerhalb des äußeren Rohrs (120) angeordnet ist, umfasst, wobei der Implantat-Zuführgriff (2) dazu konfiguriert ist, eine axiale Bewegung des äußeren und des inneren Rohrs (120, 110) anzutreiben.

9. Implantatsystem, das den Implantat-Zuführgriff (2) nach einem der Ansprüche 1 bis 7, ein äußeres Rohr (120), ein inneres Rohr (110), eine sich verjüngende Spitze (140) und eine Halterung (150) umfasst, wobei die Halterung (150) an einem distalen Ende des inneren Rohrs (110) angeordnet ist, wobei die konische Spitze (140) an einem distalen Ende des äußeren Rohrs (120) angeordnet ist, wobei ein Implantat in einem distalen Endabschnitt des äußeren Rohrs (120) zwischen der sich verjüngenden Spitze (140) und der Halterung (150) gecrimpt ist, und wobei der Implantat-Zuführgriff (2) dazu konfiguriert ist, eine axiale Bewegung des äußeren und des Inneren Rohrs (120, 110) anzutreiben, um das Laden, Zuführen und Freigeben des Implantats zu ermöglichen.

## Revendications

1. Manche de pose d'implant (2), comprenant deux sous-systèmes de transport (300, 400) agencés le long d'une direction axiale sur un tube externe (120), dans lequel : le sous-système de transport (300), situé au niveau d'une extrémité distale, est couplé à une extrémité proximale du tube externe (120) ; le sous-système de transport (400), situé au niveau d'une extrémité proximale, est couplé à une extrémité proximale d'un tube interne (110) ; et chaque sous-système de transport (300, 400) comprend un dispositif de transport synchrone (320, 420) et un ensemble d'encliquetage (340, 440) fixé au dispositif de transport synchrone (320, 420),
dans lequel l'ensemble d'encliquetage (340, 440) comprend une came (3461), une roue à rochet (342), un premier cliquet (344) et un second cliquet (345), dans lequel la roue à rochet (342) est fixée au dispositif de transport synchrone (320) et est configurée pour coopérer avec les deux cliquets (344, 345) pour commander un mouvement axial et une direction de mouvement axial du tube externe ou interne (120, 110) pour permettre une transmission de guidage autobloquante sûre du tube externe ou interne (120, 110), dans lequel : une première extrémité de chacun des deux cliquets (344, 345) est disposée de manière rotative d'un côté de la came (3461) ; une seconde extrémité de chacun des deux cliquets (344, 345) est disposée à proximité de la roue à rochet (342) ; et les deux cliquets (344, 345) sont configurés pour commander une rotation et un sens de rotation de la roue à rochet (342), dans lequel la came (3461) est disposée de manière rotative entre les deux cliquets (344, 345) et est configurée pour commander un pivotement des deux cliquets (344, 345).

2. Manche de pose d'implant (2) selon la revendication 1, dans lequel chaque sous-système de transport (300, 400) comprend en outre un mécanisme de retenue (310, 410) et un volant (330, 430), dans lequel le mécanisme de retenue (310, 410) est couplé au dispositif de transport synchrone (320, 420) et au tube externe ou interne (120, 110), dans lequel le volant (330, 430) est couplé au dispositif de transport synchrone (320, 420) et est configuré pour entraîner un mouvement axial du tube externe ou interne (120, 110) par le dispositif de transport synchrone (320, 420) et le mécanisme de retenue (310, 410), et dans lequel le volant (330, 430) et l'ensemble d'encliquetage (340, 440) sont disposés radialement sur deux côtés du dispositif de transport synchrone (320,420) respectivement.

3. Manche de pose d'implant (2) selon la revendication 2, dans lequel l'ensemble d'encliquetage (340, 440) comprend en outre un logement (341) de roue à rochet, un couvercle (347) de logement et un élément coulissant (343), dans lequel : le logement (341) de roue à rochet est disposé radialement d'un côté du mécanisme de retenue (310, 410) à l'écart du volant (330, 430) ; et le logement (341) de roue à rochet est formé dans une surface de celui-ci éloignée du mécanisme de retenue (310, 410) avec un creux (3411) dans lequel sont logé la came (3461), la roue à rochet (342) et les deux cliquets (344, 345), dans lequel le couvercle (347) de logement est disposé sur le creux (3411), l'élément coulissant (343) est inséré à travers le couvercle (347) de logement et est couplé à la came (3461), dans lequel l'élément coulissant (343) est configuré pour amener la came (3461) à pivoter, dans lequel la première extrémité de chaque cliquet (344, 345) est fixée de manière rotative à une paroi latérale du creux (3411) et une surface circonférentielle extérieure autour de la seconde extrémité de chaque cliquet (344, 345) est couplée élastiquement à la paroi latérale du creux (3411).

4. Manche de pose d'implant (2) selon la revendication 3, dans lequel la roue à rochet (342) et le volant (330, 430) sont disposés coaxialement.

5. Manche de pose d'implant (2) selon la revendication 4, dans lequel
la came (3461) appuie sur l'un des cliquets (344, 345) pour permettre une rotation unidirectionnelle de la roue à rochet (342), ou
dans lequel la came (3461) est disposée de manière espacée entre les deux cliquets (344, 345) pour limiter la rotation de la roue à rochet (342).

6. Manche de pose d'implant (2) selon la revendication 2, dans lequel le dispositif de transport synchrone (320, 420) comprend une poulie motrice (321, 421), une poulie menée (322, 422) et une bande transporteuse synchrone (323, 423) disposée sur les poulies motrice et menée (321, 421; 322, 422), dans lequel la poulie motrice (321, 421) est disposée coaxialement au volant (330, 430) et est configurée pour tourner avec le volant (330, 430) pour entraîner les poulies menée et motrice (321, 421; 322, 422) en rotation dans un même sens pour amener la bande transporteuse synchrone (323, 423) à se déplacer dans la direction axiale du tube externe ou interne (120, 110).

7. Manche de pose d'implant (2) selon la revendication 2, dans lequel le mécanisme de retenue (310, 410) comprend une partie fixe (311) de cathéter, une partie fixe (312) de plaque de serrage et une plaque de serrage (313), dans lequel la partie fixe (311) de cathéter est emmanchée sur une partie d'extrémité proximale du tube externe ou interne (120, 110), dans lequel la partie fixe (312) de plaque de serrage est fixée à la partie fixe (311) de cathéter et fait face à la bande transporteuse synchrone (323, 423), et dans lequel la plaque de serrage (313) est retenue sur la partie fixe (312) de plaque de serrage, la bande transporteuse synchrone (323, 423) étant serrée entre la plaque de serrage (313) et la partie fixe (312) de plaque de serrage.

8. Système de pose d'implant, comprenant le manche de pose d'implant (2) selon l'une quelconque des revendications 1 à 7 et un ensemble cathéter (1), dans lequel l'ensemble cathéter (1) comprend le tube externe (120) et le tube interne (110) disposé à l'intérieur du tube externe (120), dans lequel le manche de pose d'implant (2) est configuré pour entraîner un mouvement axial des tubes externe et interne (120, 110).

9. Système d'implant, comprenant le manche de pose d'implant (2) selon l'une quelconque des revendications 1 à 7, un tube externe (120), un tube interne (110), une pointe effilée (140) et un dispositif de retenue (150), dans lequel le dispositif de retenue (150) est disposé sur une extrémité distale du tube interne (110), dans lequel la pointe effilée (140) est disposée sur une extrémité distale du tube externe (120), dans lequel un implant est comprimé dans une partie d'extrémité distale du tube externe (120) entre la pointe effilée (140) et le dispositif de retenue (150), et dans lequel le manche de pose d'implant (2) est configuré pour entraîner un mouvement axial des tubes externe et interne (120, 110) afin de permettre le chargement, la pose et la libération de l'implant.
